(19) Europäisches Patentamt
European Patent Office
Office européen des brevets

(11) **EP 1 750 703 B1**

(12) **EUROPEAN PATENT SPECIFICATION**

(45) Date of publication and mention
of the grant of the patent:
**11.01.2012 Bulletin 2012/02**

(51) Int Cl.:
*A61K 31/4412* (2006.01)    *A61K 31/513* (2006.01)
*A61K 31/53* (2006.01)      *A61K 45/00* (2006.01)
*A61P 35/00* (2006.01)      *A61K 47/22* (2006.01)

(21) Application number: **05738545.2**

(22) Date of filing: **27.04.2005**

(86) International application number:
**PCT/JP2005/008450**

(87) International publication number:
**WO 2005/105086 (10.11.2005 Gazette 2005/45)**

(54) **METHOD FOR REDUCING GASTROINTESTINAL TOXICITY DUE TO THE ADMINISTRATION OF TEGAFUR**

VERFAHREN ZUR VERRINGERUNG DER GASTROINTESTINALEN TOXIZITÄT DURCH VERABREICHUNG VON TEGAFUR

PROCÉDÉ SERVANT À RÉDUIRE LA TOXICITÉ GASTRO-INTESTINALE DUE À L'ADMINISTRATION DE TÉGAFUR

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(30) Priority: **29.04.2004 US 566180 P**

(43) Date of publication of application:
**14.02.2007 Bulletin 2007/07**

(73) Proprietor: **TAIHO PHARMACEUTICAL CO., LTD.**
**Tokyo 101-0054 (JP)**

(72) Inventors:
• **TAHARA, Takeshi**
**Yokohama-shi,**
**Kanagawa 2270047 (JP)**
• **AMBE, Hiroshi**
**Shinagawa-ku, Tokyo 1400015 (JP)**
• **KURITANI, Jun**
**229159 (SG)**
• **NOMURA, Naruo**
**DECEASED (JP)**

(74) Representative: **HOFFMANN EITLE**
**Patent- und Rechtsanwälte**
**Arabellastraße 4**
**81925 München (DE)**

(56) References cited:
**EP-A1- 0 543 015**

• **PETERS G J ET AL: "The effect of food on the pharmacokinetics of S-1 after single oral administration to patients with solid tumors" CLINICAL CANCER RESEARCH 15 JUL 2004 UNITED STATES, vol. 10, no. 12 I, 15 July 2004 (2004-07-15), pages 4072-4076, XP002464280 ISSN: 1078-0432**
• **PETERS G J ET AL: "Pharmacokinetics of S-1, an oral formulation of ftorafur, oxonic acid and 5-chloro-2,4-dihydroxypyridine (molar ratio 1:0.4: 1) in patients with solid tumors" CANCER CHEMOTHERAPY AND PHARMACOLOGY 01 JUL 2003 GERMANY, vol. 52, no. 1, 1 July 2003 (2003-07-01), pages 1-12, XP002464281 ISSN: 0344-5704**
• **DAMLE B ET AL: "Effect of food on the oral bioavailability of UFT and leucovorin in cancer patients" CLINICAL CANCER RESEARCH 2001 UNITED STATES, vol. 7, no. 3, 2001, pages 517-523, XP002464282 ISSN: 1078-0432**
• **SAKATA Y. ET AL EUR J CANCER. vol. 34, no. 11, 1998, pages 1715 - 1720, XP004285120**
• **CHOLLET P. ET AL EUR J CANCER. vol. 39, no. 9, 2003, pages 1264 - 1270, XP004425218**

**Description**

TECHNICAL FIELD

The present invention relates to a method for administering Tegafur [5-Fluoro-1-[(RS)-tetrahydrofuran-2-yl] pyrimidine-2,4-(1 H,3H)-dione] to reduce gastrointestinal toxicity.

BACKGROUND ART

U.S. Patent No. 5,155,113 teaches that a kind of pyridine compound can be used as a potentiator of the antitumor effect of 5-fluorouracil and derivatives of 5-FU. The pyridine compound sustains the concentration of 5-FU in the living body. Unfortunately, the prolonged presence of 5-FU in a living body is likely to cause inflammation in the oral cavity and gastrointestinal tissue and induce diarrhea as often experienced with continuous intravenous infusion of 5-FU alone.

U.S. Patent No. 5,116,600 reports that oxonic acid used in combination with 5-FU or a 5-FU derivative can inhibit the occurrence of inflammation caused by 5-FU or a 5-FU derivative. However, oxonic acid reduces the antitumor effect of 5-FU or 5-FU derivatives. Therefore, oxonic acid does not produce satisfactory results in terms of potentiation of antitumor effect and alleviation of adverse effects.

U.S. Patent No. 5,525,603, describes compositions, methods and kits for potentiating the antitumor effect of 5-fluorouracil (hereinafter referred to as "5-FU") by using oxonic acid or a salt thereof and a compound such as 2,4-dihydroxy-5-chloropyridine or 2,4-dihydroxy-5-cyanopyridine. Though this combination reduces the inflammation caused by administration of 5-FU, severe diarrhea, vomiting and dehydration can still occur.

As shown by the above discussed patents, 5-FU and its derivatives have often been used to treat gastrointestinal (GI) cancers, breast cancer, head and neck cancer, etc. However, when administered as a single agent, the response rate to 5-FU therapy is unsatisfactory. A number of studies, including trials modifying dosing regimens, have been conducted to improve upon the efficacy and tolerability of 5-FU therapy. Lokich et al., J. Clin. Oncl., 7, 425-432 (1989) reported their attempt to improve the clinical efficacy of 5-FU (i.e., increase its antitumor activity while reducing its adverse effects). They reported that the response rate to 5-FU was higher and the incidence of myelosuppression was lower when the drug was administered by continuous venous infusion (CVI) than when it was administered by conventional bolus infusion. The usefulness of the CVI dosing regimen for 5-FU therapy has also been found in other Phase II clinical studies [Quebbeman, E. et al., J. Surg. Oncol., 30,60-65 (1985); Hansen, R. et al., J. Surg. Oncol., 40, 177-181 (1989); Caballero, G. A. et al., Cancer Treat. Rep., 69, 13-15 (1985); Barbounis, V. P. et al., Anticancer Res., 9, 33-39 (1989); Moynihan, T. et al., Am.J. Clin. Oncol., I 1, 461-464 (1988); Huan, S. et al., Cancer, 63, 419-422 (1989); Hansen, R. et al., Am. J. Med. Sci., 295, 91-93 (1988); Hansen, R. et al., Urology, 37, 358-361 (1991)]. In Japan, Yamao et al. reported the usefulness of 5-FU-CVI therapy [Yamao, T. et al., Jpn. J. Clin. Oncol., 25, 46-50 (1995)].

These results suggest that maintaining high serum 5-FU levels for prolonged periods of time after administration is a major factor determining the clinical efficacy of 5-FU therapy. However, dosing regimens of 5-FU that allow continued high blood 5-FU levels (e.g., CVI) are associated with several problems. One problem is the circadian variations in blood 5-FU levels [Petit, E. et al., Cancer Res., 48,1676-1679 (1988); Harris, B. E. et al., Cancer Res., 50, 197-201 (1990); Metzger, G. et al., Clin. Pharmacol. Ther., 56, 190-201 (1994)]. It has been reported that this problem is related to dihydropyrimidine dehydrogenase (hereinafter referred to as "DPD") and that this enzyme is an important determinant of the sensitivity of tumors to 5-FU [Fleming, R. A. et al., Cancer Res., 52, 2899-2902 (1992); Fleming, R. A. et al., Eur. J. Cancer, 29A, 740-744 (1993); Etienne, M. C. et al., J. Clin. Oncl., 12, 2248-2253 (1994); Beck, A. et al., Eur. J. Cancer, 30A, 1517-1522 (1994); Peters, G. J. et al., Eur. J. Cancer, 30A, 1408-1411 (1994); Lu, Z., et al., Cancer Res., 53, 5433-5438 (1993); Etienne, M. C. et al., J. Clin. Oncl., 13, 1663-1670 (1995)]. The second problem is the occurrence of gastrointestinal toxicity (diarrhea, nausea, vomiting, stomatitis, etc.) induced by continued high blood 5-FU levels.

Attempts have been made to prevent or reduce the adverse effects of 5-FU. In 1979, Schwartz et al., Cancer Res., 39, 3095-3101 (1979), reported that allopurinol (drug used to treat hyperuricemia) alters 5-FU and in so doing suppresses its toxic actions. Since then, clinical studies have evaluated the possibility of augmenting the antitumor activity of 5-FU and reducing its toxicity by combining 5-FU with modulators such as allopurinol [Fox, R. M. et al., Cancer Treat. Rev., 6, 143-147 (1979); Kroener, J. F. et al., Cancer Treat. Rep., 66, 1133-1137 (1982); Howell, S. B. et al., Cancer, 48, 1281-1289 (1981); Woolley, P. V. et al., J. Clin. Oncl., 3,103-109 (1985); Ahmann, F. R. et al.,Oncology, 43, 83-85 (1986)]. However, combining 5-FU with allopurinol in these clinical studies did not result in suppression of 5-FU-related toxicity or an increase in the response rate (i.e. antitumor activity). The effects of an allopurinol gargle in preventing 5-FU-induced stomatitis were also studied clinically, but sufficient evidence of such an effect was never obtained [Clark, P. I. et al., . Eur. J. Surg. Oncol., 11, 267-268 (1985); Vliet, W. et al., Clin. Pharm., 8, 655-658 (1989); Loprinzi, C. L. et al., Cancer, 65, 1879-1882 (1990)].

There are two key factors which are considered to be important in addressing the goal of making 5-FU more effective and tolerable: 1) inhibition of DPD activity so that high in vivo 5-FU levels can be maintained for long periods of time, and 2) control of 5-FU metabolism in normal tissue in order to minimize adverse events.

To achieve these goals, it was considered essential to select effectors that are retained in the blood and to use at least two effective modulators at a time. If the effector and the two modulators were administered separately, it would be difficult to achieve these goals, and the toxicity of 5-FU might be increased rather than reduced. Therefore the effector and the two modulators should be administered at the same time, so that the safety of the combination of the three drugs is ensured. Tegafur (FT), a pro-drug of 5-FU, is retained in the blood for long periods when administered orally and *in vivo* is gradually converted into 5-FU in the presence of liver P-450. In view of this, higher blood levels of 5-FU, close to the levels achieved by CVI, were expected to be obtained by oral administration of tegafur than by oral or intravenous administration of 5-FU itself. In practice, however, the blood 5-FU levels after an oral dose of tegafur were lower than expected, due to biodegradation by DPD. Combining tegafur with a powerful DPD inhibitor achieves high blood 5-FU levels for long periods at relatively low tegafur dose levels, resulting in increased antitumor activity.

DISCLOSURE OF THE INVENTION

The present invention relates to a method for potentiating the antitumor effect of tegafur while reducing gastrointestinal toxicity in a patient in need of such treatment, comprising simultaneously administering to said patient a therapeutically effective amount of tegafur, a dihydropyrimidine dehydrogenase inhibitor in an amount effective for potentiating the antitumor effect and oxonic acid or a pharmaceutically acceptable salt thereof in an amount effective for suppressing gastrointestinal toxicity, under fasting conditions. Preferably, the administrations of the composition are twice over the course of a day, under fasting conditions and the dihydropyrimidine dehydrogenase inhibitor is 2,4-dihydroxy-5-chloropyridine. The two administrations of the composition are preferably separated by 6-12 hours and the fasting conditions occur at least one hour before a meal. The 2,4-dihydroxy-5-chloropyridine and the oxonic acid or a pharmaceutically acceptable salt thereof can be used in a molar ratio of 0.4:1, preferably, tegafur, 2,4-dihydroxy-5-chloropyridine, and oxonic acid or a pharmaceutically acceptable salt thereof, are used in a molar ratio of (tegafur):(2,4-dihydroxy-5-chloropyridine):(oxonic acid or a pharmaceutically acceptable salt thereof) =1:0.4:1 and the pharmaceutically acceptable salt is potassium oxonate.

According to the present invention, tegafur is preferably administered at a dose of between 20 mg/m$^2$ to 45 mg/m$^2$ twice daily. Additional chemotherapy agents can be administered along with the tegafur. Such chemotherapy agents include cisplatin, 1,3-bis(2-chloroethyl)-1-nitrosourea, docetaxel, doxorubicin, epirubicin, etoposide, methotrexate, mitomycin, gemcitabine, carboplatin, gefitinib, pemetrexed, Avastin, Cetuximab and paclitaxel. Such chemotherapy agents, when used, are administered depending on the progress stage of cancer, prior treatment for the cancer, anticancer activity of a drug against a specific cancer type, gene expression of patients, patient's age, sex, severity of the symptoms and side effects, etc. For example, the chemotherapy agent cisplatin is preferably administered intravenously at a dose between 50-80 mg/m$^2$ daily.

The present invention can also be used for treating cancer in a mammal wherein the cancer is susceptible to 5-fluorouracil therapy, the method comprising simultaneously administering to the mammal a therapeutically effective amount of tegafur, 2,4-dihydroxy-5-chloropyridine in an amount effective for potentiating the antitumor effect of tegafur, and oxonic acid or a pharmaceutically acceptable salt thereof in an amount effective for suppressing gastrointestinal toxicity, under fasting conditions. Preferably, the administrations of the composition are twice over the course of a day, under fasting conditions and more preferably are separated by 6-12 hours, where the fasting conditions occur at least one hour before a meal. The molar ratio of (tegafur):(2,4-dihydroxy-5-chloropyridine):(oxonic acid) is preferably 1:0.4:1 and the tegafur is preferably administered in a dose of between 20 mg/m$^2$ to 45 mg/m$^2$ twice daily.

The present invention also relates to a method for inhibiting inflammation and gastrointestinal toxicity caused by the administration of tegafur, comprising administering oxonic acid or a pharmaceutically acceptable salt thereof in an amount effective for suppressing gastrointestinal toxicity, simultaneously with said tegafur to a patient in need of such treatment, wherein said tegafur and oxonic acid or a pharmaceutically acceptable salt thereof are administered under fasting conditions. Preferably the administrations of the composition are twice over the course of a day and a dihydropyrimidine dehydrogenase inhibitor is simultaneously administered in an amount effective for potentiating the antitumor effect of tegafur.

The present invention also relates to a method for decreasing the breakdown of oxonic acid in the gastrointestinal tract of a patient undergoing treatment with tegafur, comprising administering oxonic acid simultaneously with said tegafur to said patient under fasting conditions, preferably twice daily. The fasting conditions can be at least one hour before or after a meal. A dihydropyrimidine dehydrogenase inhibitor can be simultaneously administered in an amount effective for potentiating the antitumor effect of tegafur. The dihydropyrimidine dehydrogenase inhibitor is preferably 2,4-dihydroxy-5-chloropyridine.

The present invention is also directed to a kit for use in treating cancer in a mammal, comprising tegafur, 2,4-dihydroxy-5-chloropyridine, oxonic acid or a pharmaceutically acceptable salt thereof in dosages suitable for administration preferably twice a day and an additional anti-tumor agent. The additional anti-tumor agents is selected from the group consisting of cisplatin, 1,3-bis(2-chloroethyl)-1-nitrosourea, docetaxel, doxorubicin, epirubicin, etoposide, methotrexate, mitomycin, gemcitabine, carboplatin, gefitinib, pemetrexed, Avastin, Cetuximab and paclitaxel.

The present invention is also directed to a kit for use in treating cancer in a mammal, comprising tegafur, 2,4-dihydroxy-5-chloropyridine, oxonic acid or a pharmaceutically acceptable salt thereof in dosages suitable for administration preferably twice a day, and optionally an additional anti-tumor agent selected from the group consisting of cisplatin, 1,3-bis(2-chloroethyl)-1-nitrosourea, docetaxel, doxorubicin, epirubicin, etoposide, methotrexate, mitomycin, gemcitabine, carboplatin, gefitinib, pemetrexed, Avastin, Cetuximab and paclitaxel, the kit further comprising an indicator in such a form as an image printed thereon, a label attached thereto, a package insert enclosed therein or the like that reminds the patient that said tegafur, said 2,4-dihydroxy-5-chloropyridine, and said oxonic acid or a pharmaceutically acceptable salt thereof should be administered under fasting conditions.

Similarly, the present invention also provides a pharmaceutical composition for use in potentiating the antitumor effect of tegafur while reducing gastrointestinal toxicity in a patient in need of such treatment, comprising (A) a therapeutically effective amount of tegafur, (B) a dihydropyrimidine dehydrogenase inhibitor in an amount effective for potentiating the antitumor effect and (C) oxonic acid or a pharmaceutically acceptable salt thereof in an amount effective for suppressing gastrointestinal toxicity, and optionally an additional anti-tumor agent selected from the group consisting of cisplatin, 1,3-bis(2-chloroethyl)-1-nitrosourea, docetaxel, doxorubicin, epirubicin, etoposide, methotrexate, mitomycin, gemcitabine, carboplatin, gefitinib, pemetrexed, Avastin, Cetuximab and paclitaxel, the pharmaceutical composition being contained in a package that comprises an indicator in such a form as an image printed thereon, a label attached thereto or a package insert enclosed therein or the like that reminds the patient that the pharmaceutical composition should be administered under fasting conditions. The administration timing and other details are those described above and those that will be described below.

## DESCRIPTION OF THE INVENTION

The present invention relates to a method for potentiating the antitumor effect of an antitumor composition containing a therapeutically effective amount of tegafur while suppressing the gastrointestinal side effects of the antitumor composition by administering the antitumor composition under fasting conditions in order to avoid any undesirable effects of food on the bioavailability of the active components.

In a preferred embodiment of the present invention, S-1, an oral pyrimidine fluoride-derived anti-cancer agent containing Tegafur [5-Fluoro-1-[(RS)-tetrahydrofuran-2-yl]pyrimidine-2,4-(1H,3H)-dione](FT) in combination with 2 classes of modulators: 5-chloro-2,4-dihydroxypyridine (CDHP) and potassium oxonate (Oxo), is administered, typically twice a day, under fasting conditions to reduce gastrointestinal toxicity while maintaining antitumor efficacy. S-1 (as described in U.S. Patent No. 5,525,603) was developed to enhance the clinical advantage of 5-FU and ameliorate its disadvantages (i.e., gastrointestinal toxicity), and was tested by being administered after meal in the US and Europe in the same way as in Japan where S-1 was already approved, but unfortunately still produces severe diarrhea which can result in dehydration if administered under non-fasting conditions twice a day.

The purpose of combining three drugs (FT, CDHP, and Oxo) in the formulation of S-1 was to achieve high clinical efficacy by reducing the gastrointestinal toxicity and improving tolerability. The contributions of each of the three components of S-1 are outlined below:

- FT is a prodrug of 5-FU, with excellent oral bioavailability. It is gradually converted to 5-FU in vivo.
- CDHP inhibits the catabolism of 5-FU by reversibly inhibiting the enzyme of dihydropyrimidine dehydrogenase (DPD). CDHP is 180-fold more potent than uracil. CDHP helps maintain effective blood and tumor concentrations of 5-FU for a prolonged period thus potentially achieving a similar therapeutic effect as protracted infusion of 5-FU.
- Oxo blocks phosphorylation of 5-FU by orotate phosphoribosyltransferase (OPRT) in the gastrointestinal tract. Oxo shows a toxicity-reducing action on the 5-FU provoked gastrointestinal disturbances through its distribution in gastrointestinal tissues at high concentrations after oral administration. Oxo is acid labile and thus its bioavailability is affected by food intake.

Tegafur (FT) is converted to 5-FU which in itself is not cytotoxic. 5-FU has to be metabolized in the cell to exert its antitumor effects via FdUMP (2'-deoxy-5-fluorouridine-5'-monophosphate) to block TS (thymilate synthase). 90% of the administered 5-FU is rapidly catabolized mainly in the liver into inactive metabolites by the enzyme DPD. Preclinical data indicate that the 5-FU cytotoxicity can be increased significantly by blocking DPD using CDHP. Clinical data also indicate that S-1 plasma-5-FU levels are comparable to plasma-5-FU levels obtained by continuous infusion of 5-FU [Yamada Y. et al., Br. J. Cancer, 2003 Sep 1;89(5):816-20.] due to the activity of CDHP. DPD activity may be a key in the proper application of chemotherapeutics in cancer treatment. Based on a population study [Fukushima M, et al., Int. J. Mol. Med, 2003;12:839-844.] reviewing data from 2,592 clinically removed tumors (1112: colon, 724: gastric, 520:

breast and 236: NSCLC), 61 % of patients with gastric cancer, and 40% of patients with colorectal cancer were categorized as having tumors that expressed relatively high DPD activity. It is hypothesized that conventional 5-FU or its derivatives would be less effective in patients with high DPD activity than in patients with low DPD activity because 5-FU would be quickly metabolized by the high DPD activity. S-1 is expected to be effective for the treatment of gastric cancer patients not only in patients with low DPD activity, but also with high DPD activity as well due to the properties of CDHP (one component of S-1) of being a DPD inhibitor.

Without a DPD inhibitor, 90 % of 5-FU administered is rapidly catabolized by DPD primarily in the liver and excreted in the urine as F-ß-Ala (FBAL). FBAL has been implicated as the cause of neurotoxicity and cardiotoxicity associated with 5-FU administration. An important finding of DPD inhibitor studies is the low incidence of hand foot syndrome (HFS) [Milano, et al., Eur. J. Cancer., 36, 37-42 (2000)].

Accordingly the present invention relates a method for treating a cancer susceptible to 5-fluorouracil therapy in a mammal, comprising simultaneously administering to the mammal a composition comprising a therapeutically effective amount of tegafur, an antitumor effect-potentiating effective amount of a DPD inhibitor, and a side effect-suppressing effective amount of oxonic acid or a pharmaceutically acceptable salt thereof, wherein said composition is administered under fasting conditions, typically twice a day. The language "fasting conditions" as used in the present document is intended to mean at least one hour before or after a meal.

In other words, the present invention particularly provides, relates in treating a cancer susceptible to tegafur therapy in a patient in need of such treatment to a method comprising administering to the patient a therapeutically effective amount of tegafur, a DPD inhibitor, and oxonic acid or a pharmaceutically acceptable salt thereof, the improvement comprising administering the composition under fasting conditions, typically twice a day.

The tegafur for use in the present invention is a known compound and can be prepared by conventional processes as disclosed, e.g. in U.S. Patent No. 5,525,603 and Japanese Examined Patent Publication No. 10510/1974.

DPD inhibitors are known and can be easily prepared by conventional processes. Preferred DPD inhibitors include 2,4-dihydroxy-5-chloropyridine, and 2,4-dihydroxy-5-cyanopyridine.

Oxonic acid per se is a known compound. Useful pharmaceutically acceptable salts thereof include acid addition salts and basic compound salts. Examples of useful acids capable of forming the acid addition salts are hydrochloric acid, sulfuric acid, phosphoric acid, hydrobromic acid and like inorganic acids, oxalic acid, succinic acid, maleic acid, fumaric acid, malic acid, tartaric acid, citric acid, malonic acid, methanesulfonic acid, benzoic acid and like organic acids. Examples of useful basic compounds capable of forming the pharmaceutically acceptable basic compound salts are sodium hydroxide, potassium hydroxide, calcium hydroxide, sodium carbonate, potassium hydrogencarbonate, etc. Substances capable of producing oxonic acid in the living body, such as ester derivatives of oxonic acid, are usable as the oxonic acid.

Co-administration of food with S-1 was found to affect the pharmacokinetics of some of the S-1 constituents (e.g. the rate, but not the extent of absorption of tegafur and CDHP were influenced). Since toxicity of S-1 is related to the area under the curve (AUC, a measure of the total amount of drug that remains in circulation) of 5-FU [Van Groeningen C.J., Peters G.J., Schornagel J.H., Gall H., Noordhuis P., De Vries M., Turner S.L., Swart M.S., Pinedo H.M., Hanauske A.R. and Giaccone G. Phase I clinical and pharmacokinetic study of oral S-1 in patients with advanced solid tumors. J. Clin. Oncol., 18: 2772-2779, 2000], and since the bioavailability of 5-FU is within the FDA confidence intervals, it is unlikely that the safety of S-1 will be affected by food. Food did not affect the AUC of CDHP. Similarly the accumulation of uracil, an indirect result of DPD inhibition was only marginally affected. However, oxonic acid added to inhibit phosphoribosylation of 5-FU to its active metabolite FUMP (5-fluorouridine-5'-monophosphate) specifically in gut mucosa, showed a greater degradation in the co-administration of food with S-1. This indicates that it might accumulate to a lesser extent in the gut mucosa, decreasing its protective effect.

Previous studies with oral fluoropyrimidine formulations reported an increase in the time-period for absorption when food was given. Non-compartmental methods focusing on Cmax, Tmax and AUC-$_{INF}$ were used in these [Shepard D.R., Mani S., Kastrissios H., Learned-Coughlin S., Smith D., Magnum P.E.S.M., Janisch L., Fleming G.F., Schilsky R.L. and Ratain M.J. Estimation of the effect of food on the disposition of oral 5-fluorouracil in combination with eniluracil. Cancer Chemother. Pharmacol., 49: 398-402, 2002; Reigner B., Verweij J., Dirix L., Cassidy J., Twelves C., Allman D., Weidekamm E., Roos B., Banken L., Utoh M. and Osterwalder B. Effect of food on the pharmacokinetics of capecitabine and its metabolites following oral administration in cancer patients. Clin. Cancer Res., 4: 941-948, 1998] and a study [Godefridus J. Peters, Paul Noordhuis, Cornelis J. van Groeningen, Giuseppe Giaccone, Ulbe Holwerda, Daphne Voorn, Ad Schrijvers, Jan H. Schornagel, Jos H. Beijnen, Pierre Fumoleau, and Jan H. M. Schellens The Effect of food on the pharmacokinetics of S-1 after single oral administration on patients with solid tumors. Clinical Cancer Research 10: 4072-4076, 2004, published on June 15, 2004]. For capecitabine [Reigner B., Verweij J., Dirix L., Cassidy J., Twelves C., Allman D., Weidekamm E., Roos B., Banken L., Utoh M. and Osterwalder B. Effect of food on the pharmacokinetics of capecitabine and its metabolites following oral administration in cancer patients. Clin. Cancer Res., 4: 941-948, 1998] the prodrug itself and its first metabolite, showed a clear difference between the fed and fasting patients, but 5-FU was only affected marginally. In an Eniluracil (EU) -5-FU formulation, food decreased 5-FU absorption possibly because 5-FU itself was administered [Shepard D.R., Mani S., Kastrissios H., Learned-Coughlin S., Smith D., Magnum P.E.S.M., Janisch L., Fleming G.F., Schilsky R.L. and Ratain M.J. Estimation of the effect of food on the disposition of oral 5-fluorouracil in combination with eniluracil. Cancer Chemother. Pharmacol., 49: 398-402, 2002]. Irreversible suicide inactivation of DPD prevents any degradation of 5-FU; the major elimination pathway of 5-FU. Without EU the Km for elimination of 5-FU equals the Km of 5-FU for DPD [Collins J.M., Dedrick R.L., King F.G., Speyer J.L. and Myers C.E. Non-linear pharmacokinetic models for 5-fluorouracil in man. Clin. Pharmacol. Therap., 28: 235-246, 1980], but with EU this Km could not be calculated, and elimination no longer followed Michaelis-Menten kinetics anymore, leaving a linear component. With S-1, which contains a reversible DPD inhibitor, elimination of 5-FU is still biphasic, meaning that degradation still contributes to 5-FU's elimination, independent of food status.

The bioavailability of the other S-1 constituents has not previously been described. Since CDHP is not metabolised, its elimination will be by urinary excretion [Peters G.J., Noordhuis P., Van Kuilenburg A.B.P., Schornagel J.H., Gall H., Turner S.L., Swart M.S., Voorn D., Van Gennip A.H., Wanders J., Holwerda U., Smid K., Giaccone G., Fumoleau P. and Van Groeningen C.J. Pharmacokinetics of S-1, an oral formulation of tegafur, oxonic acid and 5-chloro-2,4-dihydroxypyridine (molar ratio 1:0.4:1) in patients with solid tumors. Cancer Chemother. Pharmacol., 38:1-12, 2003; Hirata K., Horikoshi N., Okazaki M., Denno R., Sasaki K., Nakano Y., Ishizuka H., Yamada Y., Uno S., Taguchi T., and Shirasaka T. Pharmacokinetic study of S-1, a novel oral fluorouracil anti-tumor drug. Clin. Cancer Res. 5: 2000-2005, 1999]. In experimental renal failure models plasma clearance of CDHP was retarded [Ikeda M., Furukawa H., Imamura H., Shimizu J., Ishida H., Masutani S., Tatsuta M., Kawasaki T. and Satomi T. Pharmacokinetic study of S-1, a novel oral fluorouracil antitumor agent in animal model and in patients with impaired renal function. Cancer Chemother. Pharmacol. 50: 25-32, 2002] in line with the degree of renal impairment. Also in patients with renal dysfunction CDHP clearance was prolonged, leading to delayed t½ and higher AUC of 5-FU [Ikeda M., Furukawa H., Imamura H., Shimizu J., Ishida H., Masutani S., Tatsuta M., Kawasaki T. and Satomi T. Pharmacokinetic study of S-1, a novel oral fluorouracil antitumor agent in animal model and in patients with impaired renal function. Cancer Chemother. Pharmacol. 50: 25-32, 2002], possibly due to a retained DPD inhibition following a prolonged exposure.

In European and US studies diarrhea was the dose-limiting toxicity in contrast to in Japan, where the formulation is registered and in clinical use with an efficacy similar to other oral formulations. Current clinical studies in the US and Europe focus on decreasing diarrhea by using another schedule such as a two-week schedule since diarrhea usually occurs in the last period of administration. Gastro-intestinal side effects of S-1 are less common in Japanese patients [Hirata K., Horikoshi N., Okazaki M., Denno R., Sasaki K., Nakano Y., Ishizuka H., Yamada Y., Uno S., Taguchi T., and Shirasaka T. Pharmacokinetic study of S-1, a novel oral fluorouracil anti-tumor drug. Clin. Cancer Res. 5: 2000-2005, 1999; Ohtsu A., Baba H. and Sakata Y. Phase II study of S-1, a novel oral fluoropyrimidine derivative, in patients with metastatic colorectal carcinoma. S-1 cooperative Colorectal Carcinoma Study Group. Br. J. Cancer, 83: 141-145, 2000] compared to Europeans [Van den Brande J., Schoffski P., Schellens J.H., Roth AD, Duffaud F., Weigang-Kohler K., Reinke F., Wanders J., De Boer R.F., Vermorken J.B. and Fumoleau P. EORTC Early Clinical Studies Group early phase II trial of S-1 in patients with advanced or metastatic colorectal cancer. Br. J. Cancer, 88: 648-53, 2003; Van Groeningen C.J., Peters G.J., Schornagel J.H., Gall H., Noordhuis P., De Vries M., Turner S.L., Swart M.S., Pinedo H.M., Hanauske A.R. and Giaccone G. Phase I clinical and pharmacokinetic study of oral S-1 in patients with advanced solid tumors. J. Clin. Oncol., 18: 2772-2779, 2000]. This might be due to potential ethnic and cultural differences (e.g. types of foods consumed, different expression of degradative enzymes, different GI absorption, etc.). Therefore, the present invention intends to decrease the gastro-intestinal side effects of S-1, and is especially useful for patients who suffer from gastro-intestinal side effects of S-1 such as diarrhea, and is typically beneficial for Caucasian patients.

Food had a profound effect on the bioavailability of oxonic acid, leading to a rapid increase in the concentration of cyanuric acid. This might decrease its anticipated protection against the gastro-intestinal side effects of 5-FU, since oxonic acid accumulates specifically in normal gut cells [Shirasaka T., Shimamoto Y. and Fukushima M. Inhibition by oxonic acid of gastrointestinal toxicity of 5-fluorouracil without loss of its antitumor activity in rats. Cancer Res., 53: 4004-4009, 1993] to prevent 5-FU metabolism to toxic metabolites. If administered along with a relatively heavy meal, protection might be limited.

The antitumor effect-potentiating method of the present invention can be carried out by formulating tegafur, the DPD inhibitor and oxonic acid or a pharmaceutically acceptable salt thereof into a single preparation (i.e. antitumor composition) and administering the composition, or by administering the separate compounds simultaneously. The combined preparation or the separate compounds are administered, typically twice daily, under fasting conditions, at least one hour and preferably 1-2 hours before a meal.

The pharmaceutical composition may be contained in a package that comprises an indicator in such a form as an image printed thereon, a label attached thereto, a package insert enclosed therein or the like that shows the timing of administration (namely, the pharmaceutical composition should be administered under fasting conditions) and optionally other information relating to the regimen described above.

Additional anti-tumor agents selected from the group consisting of cisplatin, docetaxel, paclitaxel, 1,3-bis(2-chloroethyl)-1-nitrosourea, doxorubicin, epirubicin, etoposide, methotrexate, mitomycin, gemcitabine, carboplatin, gefitinib, pemetrexed, Avastin, and Cetuximab, can be administered along with the tegafur in the present invention. S-1 has been studied in combination with cisplatin for advanced gastric cancer in Japan. The objective of the study was to identify the MTD (Maximum Tolerated Dose) of cisplatin when administered in combination with a fixed dose of S-1. The S-1 dose actually taken was similar to the late Phase II studies (mean 36.0 mg/m$^2$, range 31.8 - 39.6 mg/m$^2$) and administered twice daily for 21 days with a 14-day recovery period. Cisplatin was infused on Day 8 of a cycle at doses of 60 mg/m$^2$, or 70 mg/m$^2$. The cycle was repeated every 35 days. At the 70 mg/m$^2$ dose level, 1 patient developed Grade 4 neutropenia and another patient Grade 4 anorexia (of 6 patients). The recommended dose for the phase II portion was determined to be the first dose level of 60 mg/m$^2$. Objective responses were seen in 19 of 25 patients, evaluated yielding a 76% response rate. The PK (Pharmacokinetics) portion of the study indicates that there is no interaction between cisplatin and S-1.

Though such anti-tumor agents would be administered to the patient receiving tegafur, a DPD inhibitor, and oxonic acid or a pharmaceutically acceptable salt thereof, these agents would likely be administered at different times than the tegafur (i.e. not always simultaneously).

It is preferable to use about 0.1 to about 10 moles, preferably about 0.5 to about 5 moles, of oxonic acid or a pharmaceutically acceptable salt thereof, per mole of the DPD inhibitor for potentiating the antitumor effect of tegafur and reducing gastrointestinal toxicity. Particularly good results can be obtained by administering about 0.1 to about 5 moles, preferably about 0.1 to about 1.5 moles, of the DPD inhibitor and about 0.1 to about 5 moles, preferably about 0.2 to about 2 moles, of oxonic acid or a pharmaceutically acceptable salt thereof, per mole of tegafur. The three active ingredients can be admixed with a pharmaceutically acceptable carrier to provide a preparation in an optional unit dosage form which is then administered.

The foregoing pharmaceutical compositions are prepared in non-injection form by the conventional method using a suitable pharmaceutically acceptable carrier. Examples of useful carriers are those widely used in the manufacture of conventional pharmaceutical compositions, such as fillers, extenders, binders, disintegrators, surfactants, lubricants and like diluents and excipients. The preferred administration route is oral.

There is no particular restriction on the unit dosage form which can be adopted for the antitumor effect-potentiating composition (i.e., a composition for potentiating anti-cancer composition of tegafur, the composition comprising a DPD inhibitor such as CDHP) or antitumor composition of the invention in the treatment of malignant tumors in mammals inclusive of human beings insofar as it is in non-injection form. Thus, optional desired unit dosage form can be selected according to the purpose of treatment. Examples thereof are oral dosage forms such as tablets, coated tablets, pills, powders, granules, capsules, solutions, suspensions, emulsions, etc. and parenteral dosage forms such as suppositories, ointments, plasters, etc. These dosage forms can be manufactured by conventional pharmaceutical procedures known in this field.

As the carrier for shaping into the form of tablets, there can be employed various excipients such as lactose, sucrose, sodium chloride, glucose, urea, starch, calcium carbonate, kaolin, crystalline cellulose, silicic acid, etc.; binders such as simple syrup, glucose solution, starch solution, gelatin solution, carboxymethylcellulose, shellac, methylcellulose, potassium phosphate, polyvinylpyrrolidone, gum arabic powder, gum tragacanth powder, etc.; disintegrators such as dry starch, sodium alginate, agar powder, laminaran powder, sodium hydrogen carbonate, calcium carbonate, polyoxyethylene-sorbitan fatty acid esters, sodium lauryl sulfate, stearic acid monoglyceride, starch, lactose, etc.; antidisintegrators such as sucrose, stearic acid, cacao butter, hydrogenated oil, etc.; absorption promotors such as quaternary ammonium bases, sodium lauryl sulfate, etc.; humectants such as glycerol, starch, etc.; adsorbents such as starch, lactose, kaolin, bentonite, colloidal silicic acid, etc.; and lubricants such as purified talc, stearic acid salts, boric acid powder, polyethylene glycol, etc. Where necessary, the tablets may be in the form of coated tablets such as sugar-coated tablets, gelatin-coated tablets, enteric-coated tablets, film-coated tablets, or double or multi-layer tablets, etc.

Carriers for shaping into the form of suppositories include but are not limited to polyethylene glycol, cacao butter, higher alcohols, esters of higher alcohols, gelatin, semi-synthetic glycerides, etc.

Capsules are manufactured by mixing the antitumor composition with any of the carriers mentioned above and encapsulating the mixture in hard gelatin capsule, soft capsule or other capsules.

For manufacturing in the form of pastes, creams, and gels, there is employed a diluent such as, for example, white petrolatum, paraffin, glycerol, cellulose derivatives, polyethylene glycols, silicone, bentonite, etc.

When required, the above preparations may contain coloring agents, preservatives, perfumes, flavors, sweeteners, other medicament, etc.

The amounts of tegafur, the DPD inhibitor and oxonic acid or a pharmaceutically acceptable salt thereof, which are the active ingredients used in the invention, are dependent on the dosage form, route of administration, dosing schedule, etc. and can be appropriately chosen without specific limitation. Generally, however, the total amount of active ingredients in the dosage form may range from about 1 to about 70 percent by weight.

In the present invention, the dosage of each active ingredient can be selected according to the method of administration, the patient's age, sex and other factors, the degree of disease and so on. In the case of oral administration, the standard dosage for a human adult is usually about 0.1 to about 100 mg/kg/day, preferably about 1 to about 30 mg/kg/day, for tegafur, about 0.1 to about 100 mg/kg/day, preferably about 1 to about 50 mg/kg/day, for the DPD inhibitor and about 0.1 to about 100 mg/kg/day, preferably about 1 to about 40 mg/kg/day, for oxonic acid or a pharmaceutically acceptable salt thereof. The composition of the invention is typically administered twice daily at an interval of 6 to 12 hours under fasting conditions. In the case of suppositories, for human adults, the equivalent of about 1 to 100 mg/kg/day of tegafur is administered into the rectum twice a day at an interval of 6 to 12 hours.

In the case of oral administration of S-1 (namely, a composition comprising tegafur, CDHP and Oxo in a tegafur: CDHP:Oxo molar ratio of 1:0.4:1), the tegafur is preferably administered in a dose of between 20 $mg/m^2$ to 45 $mg/m^2$ twice daily.

The dose for a patient is usually determined based on the body surface area (BSA) calculated from the height and weight of the patient. The calculation of the body surface area is carried out using a conventional suitable method depending on the race, sex, health condition, symptom, and the like of the patient, for example, using one of the following calculating formulas under items (1) to (6) below, preferably using the calculating formula under item (2)(b) or item (6). For example, the calculating formula under item (6) below can be used for calculating BSA of Asian patients especially Japanese patients and the calculating formula under item (2) (b) below can be used for calculating BSA of Caucasian patients.

(1) The Mosteller formula (See N. Engl. J. Med. 1987 Oct 22; 317 (17): 1098 (letter))

$$BSA\ (m^2) = ([Height\ (cm) \times Weight\ (kg)]/3600)^{1/2}$$

(2) The DuBois and DuBois formula (See Arch. Int. Med. 1916 17: 863-71; J. Clin. Anesth. 1992; 4 (1): 4-10)

(a) BSA $(m^2)$ = 0.20247 $\times$ Height $(m)^{0.725}$ $\times$ Weight $(kg)^{0.425}$
(b) BSA $(m^2)$ = 0.007184 $\times$ Height $(cm)^{0.725}$ $\times$ Weight $(kg)^{0.425}$

(3) The Haycock formula (See The Journal of Pediatrics 1978 93: 1: 62-66)

$$BSA\ (m^2) = 0.024265 \times Height\ (cm)^{0.3964} \times Weight\ (kg)^{0.5378}$$

(4) The Gehan and George formula (See Cancer Chemother. Rep. 1970 54: 225-35)

$$BSA\ (m^2) = 0.0235 \times Height\ (cm)^{0.42246} \times Weight\ (kg)^{0.51456}$$

(5) The Boyd formula (See Minneapolis: University of Minnesota Press, 1935)

$$BSA\ (m^2) = 0.0003207 \times Height\ (cm)^{0.3} \times Weight\ (gram)^{(0.7285 - (0.0188 \times LOG\ (gram)))}$$

(6) The Fujimoto formula (See Nippon Eiseigaku Zasshi 1968;5:443-50)

$$BSA(cm^2) = 88.83 \times Height(cm)^{0.663} \times Weight(kg)^{0.444}$$

For example, when the body surface area of a cancer patient 175 cm high and 70 kg in weight is calculated by the above formula of item (2) (b), the area is determined to be [0.007184 $\times$ 175$(cm)^{0.725}$ $\times$ 70$(kg)^{0.425}$ = 1.85$(m^2)$.

In the case of oral administration of S-1, the standard dosage for a human adult is usually about 40 to about 300 mg/day, preferably about 50 to about 200 mg/day, for tegafur, about 10 to about 90 mg/day, preferably about 14 to about 60 mg/day, for CDHP and about 38 to about 293 mg/day, preferably about 48 to about 195 mg/day, for Oxo.

The route of administration of the antitumor composition of the present invention is not specifically limited insofar as it is non-injection route and may be, for example, intestinal, oral, rectal, stomatic, percutaneous or the like and can be selected according to the dosage form, the patient's age, sex and other factors, the severity of the symptom of the patient and so on. However, oral administration is preferred.

The tegafur, the DPD inhibitor and the oxonic acid or a pharmaceutically acceptable salt thereof, can be provided in the form of a kit. The components can be in separate containers or some or all of the components can be combined in a suitable formulation. Whether provided as separate components or as a formulation, the components are in dosage units suitable for administration typically twice a day. The kit may further comprise an indicator in such a form as an image printed thereon, a label attached thereto, package insert enclosed therein or the like that shows the timing of administration (namely, the pharmaceutical composition should be administered under fasting conditions) and optionally other information relating to the regimen described above.

Additional anti-tumor agents described above can also be included in the kit. Though such anti-tumor agents would be administered to the patient receiving tegafur, a DPD inhibitor, and oxonic acid or a pharmaceutically acceptable salt thereof, these agents would likely be administered at different times than the tegafur (i.e. not always simultaneously).

In preclinical studies, S-1 showed a tumor proliferation-inhibitory action at lower doses than other conventional pyrimidine fluoride-derived anticancer agents, such as 5-FU. In the 5-FU provoked diarrhea model in Cynomolgus monkeys and beagle dogs, S-1 showed a marked inhibitory action on the development of diarrhea when administered at specific times. On the basis of the results of the preclinical studies, S-1 is expected to be useful in the treatment of several types of malignant solid tumors. S-1 is currently being tested for the treatment of gastric, esophagus, colorectal, breast, NSCLC, head & neck, pancreatic, biliary tract, renal cell and prostate cancer in Japan. S-1 is also expected to be useful for the treatment of cancers of the liver, gallbladderbile duct, urinary bladder, uterine, cervix, and so on.

S-1 was approved for use in Japan for the treatment of gastric cancer in January 1999, in April of 2001 for head and neck cancer, in December of 2003 for colorectal cancer, and in December 2004 for NSCLC. S-1 was approved in South Korea for the treatment of gastric cancer and head and neck cancer in July 2003, and for colorectal cancer in September 2004 based on Japanese data.

The present invention will be described in more detail with reference to the following examples.

EXAMPLES

(1) <u>S-1 administered orally, twice a day, after meal</u> (comparative example)

A Phase I study has been conducted in the US under IND #53,765. This was a 35-day study (28-day treatment period, 7-day rest period). S-1 was administered orally, twice daily, after meal, for 28 consecutive days in a total of 16 patients. The dose levels were 30 mg/m$^2$, 35 mg/m$^2$, and 40 mg/m$^2$. Three patients were to be studied at each dose level before escalating doses, and an additional 10 patients were to be treated at the maximum dose in order to fully define the effects of S-1.

The antitumor effect of S-1 was determined by measuring the reduction in tumor size. A partial response was documented in one patient with colorectal cancer. Fourteen (87.5%) patients discontinued the study, the majority (11; 68.8%) from disease progression. One (6.3%) patient discontinued due to an adverse event (AE) and 2 (12.5%) patients discontinued due to unacceptable toxicity. Two (12.5%) patients died within 30 days of the last dose of S-1 and were considered to be unlikely to be related to S-1 therapy. During the study, 8 (50.0%) patients experienced serious adverse events (SAEs). Serious adverse events considered to be related to S-1 included diarrhea, dehydration, and vomiting. The MTD of S-1 was determined to be 30 mg/m$^2$. Dose-limiting toxicities (DLTs) during Course 1 included the following: diarrhea, dehydration, abdominal pain, bilirubinemia, and the inability of the patient to take =75% of the planned chemotherapy. The toxicity was reversible in almost all cases by interrupting or discontinuing S-1 therapy. At the MTD, the majority (7/9; 77.8%) of patients experienced AEs of maximum toxicity Grade 2 or lower during Course 1, showing that S-1 was well tolerated at the MTD of 30 mg/m$^2$.

(2) <u>Determination of Dose Limiting Toxicity</u>

Based on the results of Japanese studies with S-1, the first dose studied was 25 mg/m$^2$ twice daily, after meal in a phase I study in Europe. No dose-limiting toxicity occurred in six patients who were treated at this dose. Five patients were then treated with 45 mg/m$^2$. This dose was determined to be the MTD, with severe diarrhea as the main dose-limiting toxicity. Another six patients were treated with a dose of 35 mg/m$^2$; no severe toxicity was observed. In six patients on 40 mg/m$^2$, the last dose level studied, severe diarrhea was again observed (mainly in patients who had received extensive prior chemotherapy). To evaluate the safety of this dose level in patients who had not received extensive prior chemotherapy, another five patients who had not been extensively pre-treated, were included.

<Results>

Treatment with 25 mg/m$^2$ of S-1 twice daily showed mild toxicity. At 45 mg/m$^2$ twice daily, diarrhea (Grade 3 in 1 patient, Grade 4 in 2 patients) was the DLT. Other severe toxicities were Grade 4 vomiting (1 patient), Grade 3 anorexia (2 patients), Grade 3 fatigue/malaise (2 patients) and Grade 3 leukopenia/neutropenia (1 patient). No severe toxicity was observed at 35 mg/m$^2$ twice daily. At 40 mg/m$^2$, diarrhea was the DLT for patients who had received extensive prior chemotherapy: of 3 patients who had been heavily pre-treated with chemotherapy, 1 patient had Grade 3 and 1 patient had Grade 4 diarrhea. In eight patients who had received no extensive prior chemotherapy, one case of Grade 4 diarrhea and one case of Grade 3 malaise was reported. Therefore, 45 mg/m$^2$ twice daily is the MTD for patients without extensive prior chemotherapy treatment, with diarrhea as the DLT. The recommended dose for future ECSG/NDDO Phase II studies in colorectal and gastric cancer patients who have received no prior, or only 5-FU adjuvant, chemotherapy is 40 mg/m$^2$ twice daily of S-1. For patients who have been more heavily pre-treated with chemotherapy the MTD is 40 mg/m$^2$ twice daily (diarrhea as the DLT), with a recommended dose of 35 mg/m$^2$ S-1 twice daily for treatment of this category of patients.

### (3) Food Effect PK study

The effects of food on PK parameters were examined at a dose of 35 mg/m$^2$ of S-1. 17 patients were enrolled and administered S-1 both after breakfast and before breakfast in cross-over manner. The patient's diagnosis were CRC (7), breast (3), gastric (3) and other (4). The results suggested that Oxo is more stable in fasting conditions (CA, i.e., Cyanuric acid, is an inactive metabolite of Oxo). Based on this data, S-1 is administered at least 1 hour before meals in ongoing US studies.

\<Patients and methods\>

A total of 17 patients were entered in this pharmacokinetic study after informed consent. All patients were randomized for the two arms of the study and received S-1 at 35 mg/m$^2$.
In sequence A, patients received S-1 on day-7 without breakfast and on day 0 after breakfast. In sequence B, patients received S-1 after breakfast at day-7 and on day 0 without breakfast.
Blood was sampled during 24 hours. Samples were taken before and at 30 min, 1 hr, 2 hr, 4 hr, 8 hr, 10 hr and 24 hr after S-1 intake. Blood samples were taken by venipuncture in heparinized tubes of 9 ml and transported on ice for immediate processing.
Blood samples were centrifuged for 5 min at 4000 rpm and 4°C. The plasma was transferred to 2 eppendorf vials of 2 ml and stored at -20°C.
Analysis for tegafur and cyanuric acid was performed by validated HPLC assays, while the analysis of the other compounds was performed after derivatization by validated gas-chromatography-mass spectrometry methods as described previously [Peters G.J., Noordhuis P., Van Kuilenburg A.B.P., Schornagel J.H., Gall H., Turner S.L., Swart M.S., Voorn D., Van Gennip A.H., Wanders J., Holwerda U., Smid K., Giaccone G., Fumoleau P. and Van Groeningen C.J. Pharmacokinetics of S-1, an oral formulation of tegafur, oxonic acid and 5-chloro-2,4-dihydroxypyridine (molar ratio 1: 0.4:1) in patients with solid tumors. Cancer Chemother. Pharmacol., 38: 1-12, 2003.].

\<Pharmacokinetic evaluation\>

The pharmacokinetic parameters for tegafur, 5FU, oxonic acid, cyanuric acid, and CDHP were calculated with the computer program WinNonLin (version 1.5). The data were analyzed using non-compartment modeling. The parameters that could be established were: time point of maximum observed concentration in plasma (Tmax), concentration in plasma corresponding to Tmax (Cmax), terminal half-life (t½), area under the plasma concentration versus time (C-t) curve (AUCall).

### (4) RESULTS

\<Pharmacokinetics of tegafur\>

The parent compound, tegafur, reached its Tmax earlier without breakfast (Table 1). The Cmax was higher in the fasting group than the feeding group.There was no significant difference for the t½ and AUCall in either group.

\<Pharmacokinetics of 5-FU\>

The Tmax of 5-FU was reached earlier without breakfast. There was no significant difference for the t½, Cmax and AUCall in either group.

\<Pharmacokinetics of CDHP\>

The Tmax of CDHP was also reached earlier without breakfast. There was no significant difference for the t½, Cmax and AUCall in either group.

<Pharmacokinetics of Oxonic acid (Oxo)>

The Tmax of Oxonic acid was reached significantly faster without breakfast. The corresponding Cmax was significantly higher without breakfast.. The t½ was not significantly different in either group. AUCall of Oxo was higher in the fasting group than the feeding group.

<Pharmacokinetics of Cyanuric acid (CA)>

Cyanuric acid showed a faster Tmax without breakfast. The corresponding Cmax was not significantly different in either group. Also the t½ was not significantly different in either group. AUCall of cyanuric acid showed higher values in the feeding group as compared to the fasting group.

Table 1: Pharmacokinetic parameters of each analyte after oral administration of S-1 to fasting or feeding patients.

| | | AUCali (hr·ng/mL) | | Cmax (ng/mL) | | Tmax (hr) | | t½ (hr) | |
|---|---|---|---|---|---|---|---|---|---|
| | | fasted | fed | fasted | fed | fasted | fed | fasted | fed |
| FT | Mean | 15737.8 | 14817.7 | 2374.1 | 1457.2 | 0.6 | 2.1 | 8.6 | 8.6 |
| | SD | 3696.6 | 3092.1 | 628.6 | 288.1 | 0.2 | 0.8 | 1.7 | 3.2 |
| | Count | 17 | 17 | 17 | 17 | 17 | 17 | 17 | 17 |
| 5-FU | Mean | 1099.7 | 1374.6 | 210.2 | 315.2 | 1.8 | 2.7 | 2.2 | 2.6 |
| | SD | 268.2 | 1623.0 | 53.0 | 525.4 | 0.5 | 1.1 | 1.7 | 1.9 |
| | Count | 17 | 17 | 17 | 17 | 17 | 17 | 17 | 16 |
| CDHP | Mean | 1919.4 | 1685.4 | 464.0 | 328.1 | 1.1 | 2.2 | 4.5 | 5.2 |
| | SD | 380.8 | 828.3 | 121.4 | 260.9 | 0.4 | 0.9 | 2.2 | 3.3 |
| | Count | 17 | 17 | 17 | 17 | 17 | 17 | 17 | 17 |
| Oxo | Mean | 808.7 | 349.9 | 158.1 | 62.3 | 1.6 | 2.4 | 3.9 | 4.2 |
| | SD | 341.9 | 165.5 | 73.2 | 36.2 | 0.5 | 0.9 | 2.0' | 4.7 |
| | Count | 17 | 17 | 17 | 17 | 17 | 17 | 17 | 17 |
| CA | Mean | 517.3 | 1220.9 | 117.8 | 160.2 | 1.3 | 3.5 | 5.3 | 6.7 |
| | SD | 571.6 | 818.4 | 129.2 | 119.6 | 1.0 | 1.0 | 6.3 | 7.2 |
| | Count | 17 | 17 | 17 | 17 | <u>14</u> | <u>15</u> | 12 | 14 |
| CA: cyanuric acid | | | | | | | | | |

Patients were treated with 35 mg/m$^2$ in fasted or fed condition. The results summarized in Table 1 show better absorption of each S-1 component and increased 5-FU levels due to higher absorption of CDHP (DPD inhibitor) when administered before meals.
The results also suggest that Oxo is more stable in fasting conditions (CA is an inactive metabolite of Oxo). Therefore, under fasting conditions, it is expected that Oxo shows a toxicity-reducing action on the 5-FU provoked gastrointestinal disturbances satisfactorily.
Based on this data, S-1, when administered under fasting conditions, achieves excellent advantages.

INDUSTRIAL APPLICABILITY

According to the invention, excellent advantages, such as decreased gastrointestinal toxicity and diarrhea, compared to the treatment schedule of after meal administration, longer term treatment due to lower incidence of diarrhea, are achieved by simultaneously administering to a patient (A) a therapeutically effective amount of tegafur, (B) a dihydropyrimidine dehydrogenase inhibitor in an amount effective for potentiating the antitumor effect and (C) oxonic acid or a pharmaceutically acceptable salt thereof in an amount effective for suppressing gastrointestinal toxicity, under fasting conditions.

**Claims**

1. A composition comprising (A) a therapeutically effective amount of tegafur, (B) a dihydropyrimidine dehydrogenase inhibitor in an amount effective for potentiating the antitumor effect and (C) oxonic acid or a pharmaceutically acceptable salt thereof in an amount effective for suppressing gastrointestinal toxicity for use in the treatment of a patient suffering from cancer susceptible of being treated with 5-FU, wherein the treatment is carried out under fasting conditions that occur at least one hour before a meal, wherein the above components are simultaneously administered, wherein 0.1 to 5 mol of a dihydropyrimidine dehydrogenase inhibitor and 0.1 to 5 mol of oxonic acid or a pharmaceutically acceptable salt thereof per 1 mol of tegafur are administered and wherein the antitumor effect of tegafur is potentiated while reducing gastrointestinal toxicity in the patient in need of such treatment.

2. Composition for use according to claim 1, wherein the administrations of the composition are twice over the course of a day, under fasting conditions.

3. Composition for use according to claim 1, wherein the dihydropyrimidine dehydrogenase inhibitor is 2,4-dihydroxy-5-chloropyridine.

4. Composition for use according to claim 1, wherein the two administrations of the composition are separated by 6 - 12 hours.

5. Composition for use according to claim 3, wherein the 2,4-dihydroxy-5-chloropyridine (B) and the oxonic acid or a pharmaceutically acceptable salt thereof (C) are used in a molar ratio of (B) : (C) = 0.4 : 1.

6. Composition for use according to claim 1, wherein the pharmaceutically acceptable salt is potassium oxonate.

7. Composition for use according to claim 3, wherein the tegafur, the 2,4-dihydroxy-5-chloropyridine and the oxonic acid or a pharmaceutically acceptable salt thereof are used in a molar ratio of (tegafur):(2,4-dihydroxy-5-chloropyridine):(oxonic acid or a pharmaceutically acceptable salt thereof) = 1 : 0.4 : 1.

8. Composition for use according to claim 1, wherein the tegafur is administered at a dose of between 20 mg/m$^2$ to 45 mg/m$^2$ twice daily.

9. Composition for use according to claim 1, wherein a chemotherapy agent selected from the group consisting of cisplatin, 1,3-bis(2-chloroethyl)-1-nitrosourea, docetaxel, doxorubicin, epirubicin, etoposide, methotrexate, mitomycin, gemcitabine, carboplatin, gefitinib, pemetrexed, Avastin, Cetuximab and paclitaxel is further administered.

10. Composition for use according to claim 9, wherein the chemotherapy agent is cisplatin administered intravenously.

11. Composition for use according to claim 10, wherein the cisplatin is administered at a dose between 50 - 80 mg/m$^2$ daily.

12. A composition comprising a therapeutically effective amount of tegafur, 2,4-dihydroxy-5-chloropyridine in an amount effective for potentiating the antitumor effect of tegafur and oxonic acid or a pharmaceutically acceptable salt thereof in an amount effective for suppressing gastrointestinal toxicity for use in the treatment of a mammal suffering from cancer susceptible of being treated with 5-FU, wherein the treatment is carried out under fasting conditions that occur at least one hour before a meal, wherein the above components are simultaneously administered and wherein 0.1 to 5 mol of 2,4-dihydroxy-5-chloropyridine and 0.1 to 5 mol of oxonic acid or a pharmaceutically acceptable salt thereof per 1 mol of tegafur are administered.

13. Composition for use according to claim 12, wherein the administrations are twice over the course of a day, under fasting conditions.

14. Composition for use according to claim 12, wherein the two administrations are separated by 6 - 12 hours.

15. Composition for use according to claim 12, wherein the molar ratio of (tegafur):(2,4-dihydroxy-5-chloropyridine):(oxonic acid or a pharmaceutically acceptable salt thereof) is 1 : 0.4 : 1.

16. Composition for use according to claim 12, wherein the tegafur is administered at a dose of between 20 mg/m$^2$ to 45 mg/m$^2$ twice daily.

17. A composition comprising oxonic acid or a pharmaceutically acceptable salt thereof in an amount effective for suppressing gastrointestinal toxicity and tegafur for use in the treatment of a patient suffering from cancer susceptible of being treated with 5-FU, wherein the treatment is carried out under fasting conditions that occur at least one hour before a meal, wherein the above components are simultaneously administered, wherein 0.1 to 5 mol of oxonic acid or a pharmaceutically acceptable salt thereof per 1 mol of tegafur are administered and wherein inflammation and gastrointestinal toxicity caused by the administration of tegafur are inhibited.

18. A composition comprising oxonic acid or a pharmaceutically acceptable salt thereof in an amount effective for suppressing gastrointestinal toxicity and tegafur for use in inhibiting inflammation and gastrointestinal toxicity caused by the administration of tegafur, wherein oxonic acid or a pharmaceutically acceptable salt thereof and tegafur are simultaneously administered to a patient suffering from cancer susceptible of being treated with 5-FU, wherein the administration is carried out under fasting conditions that occur at least one hour before a meal and wherein 0.1 to 5 mol of oxonic acid or a pharmaceutically acceptable salt thereof per 1 mol of tegafur are administered.

19. Composition for use according to claim 17 or 18, wherein the administrations of the composition are twice a day under fasting conditions.

20. Composition for use according to claim 17 or 18, wherein a dihydropyrimidine dehydrogenase inhibitor in an amount effective for potentiating the antitumor effect of tegafur is further simultaneously administered.

21. A composition comprising oxonic acid and tegafur for use in the treatment of a patient suffering from cancer susceptible of being treated with 5-FU and undergoing treatment with tegafur, wherein the treatment is carried out under fasting conditions that occur at least one hour before a meal, wherein the above components are simultaneously administered, wherein 0.1 to 5 mol of oxonic acid per 1 mol of tegafur are administered and wherein the breakdown of oxonic acid in the gastrointestinal tract of the patient is decreased.

22. A composition comprising oxonic acid and tegafur for use in decreasing the breakdown of oxonic acid in the gastrointestinal tract of a patient suffering from cancer susceptible of being treated with 5-FU and undergoing treatment with tegafur, wherein oxonic acid is simultaneously administered with said tegafur to the patient, wherein the administration is carried out under fasting conditions that occur at least one hour before a meal and wherein 0.1 to 5 mol of oxonic acid per 1 mol of tegafur are administered.

23. Composition for use according to claim 21 or 22, wherein the administrations of tegafur simultaneously with oxonic acid are twice daily under fasting conditions.

24. Composition for use according to claim 21 or 22, wherein a dihydropyrimidine dehydrogenase inhibitor in an amount effective for potentiating the antitumor effect of tegafur is further simultaneously administered.

25. Composition for use according to claim 24, wherein the dihydropyrimidine dehydrogenase inhibitor is 2,4-dihydroxy-5-chloropyridine.

26. A kit comprising tegafur, 2,4-dihydroxy-5-chloropyridine, oxonic acid or a pharmaceutically acceptable salt thereof in dosages suitable for administration twice a day for use in treating cancer in a mammal suffering from cancer susceptible of being treated with 5-FU, the kit optionally comprising an anti-tumor agent selected from the group consisting of cisplatin, 1,3-bis(2-chloroethyl)-1-nitrosourea, docetaxel, doxorubicin, epirubicin, etoposide, methotrexate, mitomycin, gemcitabine, carboplatin, gefitinib, pemetrexed, Avastin, Cetuximab and paclitaxel, and the kit further comprising an indicator that reminds that the tegafur, the 2,4-dihydroxy-5-chloropyridine and the oxonic acid or a pharmaceutically acceptable salt thereof should be administered under fasting conditions that occur at least one hour before a meal, wherein 0.1 to 5 mol of 2,4-dihydroxy-5-chloropyridine and 0.1 to 5 mol of oxonic acid or a pharmaceutically acceptable salt thereof per 1 mol of tegafur are administered.

27. A pharmaceutical composition comprising (A) a therapeutically effective amount of tegafur, (B) a dihydropyrimidine dehydrogenase inhibitor in an amount effective for potentiating the antitumor effect and (C) oxonic acid or a pharmaceutically acceptable salt thereof in an amount effective for suppressing gastrointestinal toxicity for use in potentiating the antitumor effect of tegafur while reducing gastrointestinal toxicity in a patient suffering from cancer susceptible of being treated with 5-FU in need of such treatment, wherein 0.1 to 5 mol of a dihydropyrimidine dehydrogenase inhibitor and 0.1 to 5 mol of oxonic acid or a pharmaceutically acceptable salt thereof per 1 mol of tegafur are administered, wherein the pharmaceutical composition optionally comprises an additional antitumor agent selected from the group consisting of cisplatin, 1,3-bis(2-chloroethyl)-1-nitrosourea, docetaxel, doxorubicin, epirubicin, etoposide, methotrexate, mitomycin, gemcitabine, carboplatin, gefitinib, pemetrexed, Avastin, Cetuximab and paclitaxel, and wherein the pharmaceutical composition is contained in a package that comprises an indicator that reminds the patient that the pharmaceutical composition should be administered under fasting conditions that occur at least one hour before a meal.

**Patentansprüche**

1. Zusammensetzung, umfassend (A) eine therapeutisch wirksame Menge Tegafur, (B) einen Dihydropyrimidindehydrogenaseinhibitor in einer Menge, die zur Potenzierung der Antitumorwirkung wirksam ist, und (C) Oteracil oder ein pharmazeutisch akzeptables Salz hiervon in einer Menge, die zur Unterdrückung der gastrointestinalen Toxizität wirksam ist, zur Anwendung bei der Behandlung eines Patienten, der an Krebs leidet, der auf eine Behandlung mit 5-FU anspricht, wobei die Behandlung im nüchternen Zustand wenigstens ein Stunde vor einer Mahlzeit durchgeführt wird, wobei die oben genannten Komponenten gleichzeitig verabreicht werden, wobei 0,1 bis 5 mol eines Dihydropyrimidindehydrogenaseinhibitors und 0,1 bis 5 mol Oteracil oder ein pharmazeutisch akzeptables Salz hiervon pro 1 mol Tegafur verabreicht werden und wobei die Antitumorwirkung von Tegafur potenziert wird, während die gastrointestinale Toxizität bei einem Patienten, der einer solchen Behandlung bedarf, reduziert wird.

2. Zusammensetzung zur Anwendung gemäß Anspruch 1, wobei die Zusammensetzung im nüchternen Zustand zweimal im Laufe eines Tages verabreicht wird.

3. Zusammensetzung zur Anwendung gemäß Anspruch 1, wobei der Dihydropyrimidindehydrogenaseinhibitor 2,4-Dihydroxy-5-chlorpyridin ist.

4. Zusammensetzung zur Anwendung gemäß Anspruch 1, wobei die beiden Verabreichungen der Zusammensetzung in Abständen von 6 bis 12 Stunden erfolgen.

5. Zusammensetzung zur Anwendung gemäß Anspruch 3, wobei 2,4-Dihydroxy-5-chlorpyridin (B) und Oteracil oder ein pharmazeutisch akzeptables Salz hiervon (C) in einem Molverhältnis von (B) : (C) = 0,4 : 1 eingesetzt werden.

6. Zusammensetzung zur Anwendung gemäß Anspruch 1, wobei das pharmazeutisch akzeptable Salz Oteracil Kalium ist.

7. Zusammensetzung zur Anwendung gemäß Anspruch 3, wobei Tegafur, 2,4-Dihydroxy-5-chlorpyridin und Oteracil oder ein pharmazeutisch akzeptables Salz hiervon in einem Molverhältnis von (Tegafur):(2,4-Dihydroxy-5-chlorpyridin): (Oteracil oder ein pharmazeutisch akzeptables Salz hiervon) = 1 : 0,4 : 1 eingesetzt werden.

8. Zusammensetzung zur Anwendung gemäß Anspruch 1, wobei Tegafur zweimal täglich in einer Dosierung von 20 mg/m$^2$ bis 45 mg/m$^2$ verabreicht wird.

9. Zusammensetzung zur Anwendung gemäß Anspruch 1, wobei zusätzlich ein Chemotherapeutikum ausgewählt aus der Gruppe bestehend aus Cisplatin, 1,3-Bis(2-chlorethyl)-1-nitrosoharnstoff, Docetaxel, Doxorubicin, Epirubicin, Etoposid, Methotrexat, Mitomycin, Gemcitabin, Carboplatin, Gefitinib, Pemetrexed, Avastin, Cetuximab und Paclitaxel verabreicht wird.

10. Zusammensetzung zur Anwendung gemäß Anspruch 9, wobei das Chemotherapeutikum intravenös verabreichtes Cisplatin ist.

11. Zusammensetzung zur Anwendung gemäß Anspruch 10, wobei Cisplatin täglich in einer Dosierung von 50 bis 80 mg/m$^2$ verabreicht wird.

12. Zusammensetzung, umfassend eine therapeutisch wirksame Menge von Tegafur, 2,4-Dihydroxy-5-chlorpyridin in einer Menge, die zur Potenzierung der Antitumorwirkung von Tegafur wirksam ist, und Oteracil oder ein pharmazeutisch akzeptables Salz hiervon in einer Menge, die zur Unterdrückung der gastrointestinalen Toxizität wirksam ist, zur Anwendung bei der Behandlung eines Säugetieres, das an Krebs leidet, der auf eine Behandlung mit 5-FU anspricht, wobei die Behandlung im nüchternen Zustand wenigstens ein Stunde vor einer Mahlzeit durchgeführt wird, wobei die oben genannten Komponenten gleichzeitig verabreicht werden und wobei 0,1 bis 5 mol 2,4-Dihydroxy-5-chlorpyridin und 0,1 bis 5 mol Oteracil oder ein pharmazeutisch akzeptables Salz hiervon pro 1 mol Tegafur verabreicht werden.

13. Zusammensetzung zur Anwendung gemäß Anspruch 12, wobei die Verabreichungen im nüchternen Zustand zweimal im Laufe eines Tages stattfinden.

14. Zusammensetzung zur Anwendung gemäß Anspruch 12, wobei die beiden Verabreichungen der Zusammensetzung in Abständen von 6 bis 12 Stunden erfolgen.

15. Zusammensetzung zur Anwendung gemäß Anspruch 12, wobei das Molverhältnis von (Tegafur):(2,4-Dihydroxy-5-chlorpyridin):(Oteracil oder ein pharmazeutisch akzeptables Salz hiervon) = 1 : 0,4 : 1 beträgt.

16. Zusammensetzung zur Anwendung gemäß Anspruch 12, wobei Tegafur zweimal täglich in einer Dosierung von 20 mg/m$^2$ bis 45 mg/m$^2$ verabreicht wird.

17. Zusammensetzung, umfassend Oteracil oder ein pharmazeutisch akzeptables Salz hiervon in einer Menge, die zur Unterdrückung der gastrointestinalen Toxizität wirksam ist, und Tegafur zur Anwendung bei der Behandlung eines Patienten, der an Krebs leidet, der auf eine Behandlung mit 5-FU anspricht, wobei die Behandlung im nüchternen Zustand wenigstens ein Stunde vor einer Mahlzeit durchgeführt wird, wobei die oben genannten Komponenten gleichzeitig verabreicht werden, wobei 0,1 bis 5 mol Oteracil oder ein pharmazeutisch akzeptables Salz hiervon pro 1 mol Tegafur verabreicht werden und wobei durch die Verabreichung von Tegafur verursachten Entzündungen und gastrointestinale Toxizität gehemmt werden.

18. Zusammensetzung, umfassend Oteracil oder ein pharmazeutisch akzeptables Salz hiervon in einer Menge, die zur Unterdrückung der gastrointestinalen Toxizität wirksam ist, und Tegafur zur Anwendung bei der Hemmung von durch die Verabreichung von Tegafur verursachten Entzündungen sowie gastrointestinale Toxizität, wobei Oteracil oder ein pharmazeutisch akzeptables Salz hiervon und Tegafur gleichzeitig an einen Patienten, der an Krebs leidet, der auf eine Behandlung mit 5-FU anspricht, wobei die Behandlung im nüchternen Zustand wenigstens ein Stunde vor einer Mahlzeit durchgeführt wird, und wobei 0,1 bis 5 mol Oteracil oder ein pharmazeutisch akzeptables Salz hiervon pro 1 mol Tegafur verabreicht werden.

19. Zusammensetzung zur Anwendung gemäß Anspruch 17 oder 18, wobei die Zusammensetzung zweimal pro Tag in nüchternem Zustand verabreicht wird.

20. Zusammensetzung zur Anwendung gemäß Anspruch 17 oder 18, wobei gleichzeitig ein Dihydropyrimidindehydrogenaseinhibitor in einer Menge, die zur Verstärkung der Antitumorwirkung von Tegafur wirksam ist, verabreicht wird.

21. Zusammensetzung, umfassend Oteracil und Tegafur zur Anwendung bei der Behandlung eines Patienten, der an Krebs leidet, der auf eine Behandlung mit 5-FU anspricht und mit Tegafur behandelt wird, wobei die Behandlung im nüchternen Zustand wenigstens ein Stunde vor einer Mahlzeit durchgeführt wird, wobei die oben genannten Komponenten gleichzeitig verabreicht werden, wobei 0,1 bis 5 mol Oteracil pro 1 mol Tegafur verabreicht werden und wobei der Abbau von Oteracil im Gastrointestinaltrakt des Patienten verringert wird.

22. Zusammensetzung, umfassend Oteracil und Tegafur zur Anwendung bei der Verringerung des Abbaus von Oteracil im Gastrointestinaltrakt eines Patienten, der an Krebs leidet, der auf eine Behandlung mit 5-FU anspricht und mit Tegafur behandelt wird, wobei dem Patienten gleichzeitig mit Tegafur Oteracil verabreicht wird, wobei die Behandlung im nüchternen Zustand wenigstens ein Stunde vor einer Mahlzeit durchgeführt wird, und wobei 0,1 bis 5 mol Oteracil pro 1 mol Tegafur verabreicht wird.

23. Zusammensetzung zur Anwendung gemäß Anspruch 21 oder 22, wobei Tegafur gleichzeitig mit Oteracil zweimal täglich in nüchternem Zustand verabreicht wird.

24. Zusammensetzung zur Anwendung gemäß Anspruch 21 oder 22, wobei außerdem gleichzeitig ein Dihydropyrimidindehydrogenaseinhibitor in einer Menge, die zur Potenzierung der Antitumorwirkung von Tegafur wirksam ist, verabreicht wird.

**25.** Zusammensetzung zur Anwendung gemäß Anspruch 24, wobei der Dihydropyrimidindehydrogenaseinhibitor 2,4-Dihydroxy-5-chlorpyridin ist.

**26.** Kit, umfassend Tegafur, 2,4-Dihydroxy-5-chlorpydridin, Oteracil oder ein pharmazeutisch akzeptables Salz hiervon in Dosierungen, die zur zweimaligen Verabreichung pro Tag geeignet sind, zur Anwendung bei der Behandlung von Krebs in Säugetieren, die an Krebs leiden, der auf eine Behandlung mit 5-FU anspricht, wobei das Kit gegebenenfalls einen Antitumorwirkstoff ausgewählt aus der Gruppe bestehend aus Cisplatin, 1,3-Bis(2-chlorethyl)-1-nitrosoharnstoff, Docetaxel, Doxorubicin, Epirubicin, Etoposid, Methotrexat, Mitomycin, Gemcitabin, Carboplatin, Gefitinib, Pemetrexed, Avastin, Cetuximab und Paclitaxel umfasst, und das Kit außerdem einen Hinweis umfasst, der daran erinnert, dass Tegafur, 2,4-Dihydroxy-5-chlorpyridin und Oteracil oder ein pharmazeutisch akzeptables Salz hiervon im nüchternen Zustand wenigstens eine Stunde vor einer Mahlzeit verabreicht werden sollen, wobei 0,1 bis 5 mol 2,4-Dihydroxy-5-chlorpyridin und 0,1 bis 5 mol Oteracil oder ein pharmazeutisch akzeptables Salz hiervon pro 1 mol Tegafur verabreicht werden.

**27.** Pharmazeutische Zusammensetzung, umfassend (A) eine therapeutisch wirksame Menge Tegafur, (B) einen Dihydropyrimidindehydrogenaseinhibitor in einer Menge, die zur Potenzierung der Antitumorwirkung wirksam ist, und (C) Oteracil oder ein pharmazeutisch akzeptables Salz hiervon in einer Menge, die zur Unterdrückung der gastrointestinalen Toxizität wirksam ist, zur Anwendung bei der Potenzierung der Antitumorwirkung von Tegafur unter gleichzeitiger Verringerung der gastrointestinale Toxizität bei einem Patienten, der an einem Krebs leidet, der auf die Behandlung mit 5-FU anspricht und der einer solchen Behandlung bedarf, wobei 0,1 bis 5 mol eines Dihydropyrimidindehydrogenaseinhibitors und 0,1 bis 5 mol Oteracil oder ein pharmazeutisch akzeptables Salz hiervon pro 1 mol Tegafur verabreicht werden, und wobei die pharmazeutische Zusammensetzung gegebenenfalls einen zusätzlichen Antitumorwirkstoff ausgewählt aus der Gruppe bestehend aus aus Cisplatin, 1,3-Bis(2-chlorethyl)-1-nitrosoharnstoff, Docetaxel, Doxorubicin, Epirubicin, Etoposid, Methotrexat, Mitomycin, Gemcitabin, Carboplatin, Gefitinib, Pemetrexed, Avastin, Cetuximab und Paclitaxel umfasst, und wobei die pharmazeutische Zusammensetzung in einer Packung enthalten ist, die einen Hinweis umfasst, der den Patienten daran erinnert, dass die pharmazeutische Zusammensetzung in nüchternem Zustand wenigstens eine Stunde vor einer Mahlzeit verabreicht werden soll.

**Revendications**

**1.** Composition comprenant (A) une quantité thérapeutiquement efficace de tégafur, (B) un inhibiteur de la dihydropyrimidine déshydrogénase en quantité efficace pour potentialiser l'effet antitumoral et (C) de l'acide oxonique ou un de ses sels pharmaceutiquement acceptables en quantité efficace pour supprimer la toxicité gastro-intestinale destinée à être utilisée pour traiter un patient atteint d'un cancer susceptible d'être traité avec du 5-FU, dans laquelle le traitement est effectué en étant à jeun au moins une heure avant chaque repas, dans laquelle les composants ci-dessus sont administrés simultanément, dans laquelle on administre 0,1 à 5 moles d'inhibiteur de la dihydropyrimidine déshydrogénase et 0,1 à 5 moles d'acide oxonique ou d'un de ses sels pharmaceutiquement acceptables pour 1 mole de tégafur et dans laquelle l'effet antitumoral du tégafur est potentialisé tout en réduisant la toxicité gastro-intestinale chez le patient nécessitant ce type de traitement.

**2.** Composition destinée à être utilisée selon la revendication 1, dans laquelle la composition est administrée deux fois par jour à jeun.

**3.** Composition destinée à être utilisée selon la revendication 1, dans laquelle l'inhibiteur de la dihydropyrimidine déshydrogénase est la 2,4-dihydroxy-5-chloropyridine.

**4.** Composition destinée à être utilisée selon la revendication 1, dans laquelle les deux administrations de la composition sont espacées de 6 à 12 heures.

**5.** Composition destinée à être utilisée selon la revendication 3, dans laquelle la 2,4-dihydroxy-5-chloropyridine (B) et l'acide oxonique ou un de ses sels pharmaceutiquement acceptables (C) sont utilisés selon un rapport molaire (B)/(C) = 0,4/1.

**6.** Composition destinée à être utilisée selon la revendication 1, dans laquelle le sel pharmaceutiquement acceptable est l'oxonate de potassium.

**7.** Composition destinée à être utilisée selon la revendication 3, dans laquelle le tégafur, la 2, 4-dihydroxy-5-chloropyridine et l'acide oxonique ou un de ses sels pharmaceutiquement acceptables sont utilisés selon un rapport molaire (tégafur)/ (2,4-dihydroxy-5-chloropyridine)/(acide oxonique ou un de ses sels pharmaceutiquement acceptables) = 1/0,4/1.

**8.** Composition destinée à être utilisée selon la revendication 1, dans laquelle le tégafur est administré à une dose comprise entre 20 mg/m$^2$ et 45 mg/m$^2$ deux fois par jour.

**9.** Composition destinée à être utilisée selon la revendication 1, dans laquelle on administre en outre un agent chimiothérapique choisi dans le groupe constitué du cisplatine, de la 1,3-bis(2-chloroéthyl)-1-nitrosourée, du docétaxel, de la doxorubicine, de l'épirubicine, de l'étoposide, du méthotrexate, de la mitomycine, de la gemcitabine, du carboplatine, du géfitinib, du pemetrexed, de l'Avastin, du cetuximab et du paclitaxel.

**10.** Composition destinée à être utilisée selon la revendication 9, dans laquelle l'agent chimiothérapique est la cisplatine administrée par voie intraveineuse.

**11.** Composition destinée à être utilisée selon la revendication 10, dans laquelle la cisplatine est administrée à une dose quotidienne comprise entre 50 et 80 mg/m$^2$.

**12.** Composition comprenant une quantité thérapeutiquement efficace de tégafur, de la 2,4-dihydroxy-5-chloropyridine en quantité efficace pour potentialiser l'effet antitumoral du tégafur et de l'acide oxonique ou un de ses sels pharmaceutiquement acceptables en quantité efficace pour supprimer la toxicité gastro-intestinale destinée à être utilisée pour traiter un mammifère atteint d'un cancer susceptible d'être traité avec du 5-FU, dans laquelle le traitement est effectué en étant à jeun au moins une heure avant chaque repas, dans laquelle les composants ci-dessus sont administrés simultanément et dans laquelle on administre 0,1 à 5 moles de 2,4-dihydroxy-5-chloropyridine et 0,1 à 5 moles d'acide oxonique ou d'un de ses sels pharmaceutiquement acceptables pour 1 mole de tégafur.

**13.** Composition destinée à être utilisée selon la revendication 12, dans laquelle l'administration s'effectue deux fois par jour à jeun.

**14.** Composition destinée à être utilisée selon la revendication 12, dans laquelle les deux administrations sont espacées de 6 à 12 heures.

**15.** Composition destinée à être utilisée selon la revendication 12, dans laquelle le rapport molaire (tégafur)/(2,4-dihydroxy-5-chloropyridine)/ (acide oxonique ou un de ses sels pharmaceutiquement acceptables) est de 1/0,4/1.

**16.** Composition destinée à être utilisée selon la revendication 12, dans laquelle le tégafur est administré à une dose comprise entre 20 mg/ m$^2$ et 45 mg/m$^2$ deux fois par jour.

**17.** Composition comprenant de l'acide oxonique ou un de ses sels pharmaceutiquement acceptables en quantité efficace pour supprimer la toxicité gastro-intestinale et du tégafur destinée à être utilisée pour traiter un patient atteint d'un cancer susceptible d'être traité avec du 5-FU, dans laquelle le traitement est effectué en étant à jeun au moins une heure avant chaque repas, dans laquelle les composants ci-dessus sont administrés simultanément, dans laquelle on administre 0,1 à 5 moles d'acide oxonique ou d'un de ses sels pharmaceutiquement acceptables pour 1 mole de tégafur et dans laquelle l'inflammation et la toxicité gastro-intestinale causées par l'administration du tégafur sont inhibées.

**18.** Composition comprenant de l'acide oxonique ou un de ses sels pharmaceutiquement acceptables en quantité efficace pour supprimer la toxicité gastro-intestinale et du tégafur destinée à être utilisée pour inhiber l'inflammation et la toxicité gastro-intestinale causées par l'administration du tégafur, dans laquelle l'acide oxonique ou un de ses sels pharmaceutiquement acceptables et le tégafur sont administrés simultanément à un patient atteint d'un cancer susceptible d'être traité avec du 5-FU, dans laquelle l'administration est effectuée en étant à jeun au moins une heure avant chaque repas et dans laquelle on administre 0,1 à 5 moles d'acide oxonique ou d'un de ses sels pharmaceutiquement acceptables pour 1 mole de tégafur.

**19.** Composition destinée à être utilisée selon la revendication 17 ou 18, dans laquelle la composition est administrée deux fois par jour à jeun.

**20.** Composition destinée à être utilisée selon la revendication 17 ou 18, dans laquelle on administre en plus simultanément un inhibiteur de la dihydropyrimidine déshydrogénase en quantité efficace pour potentialiser l'effet antitumoral du tégafur.

**21.** Composition comprenant de l'acide oxonique et du tégafur destinée à être utilisée pour traiter un patient atteint d'un cancer susceptible d'être traité avec du 5-FU et en cours de traitement avec du tégafur, dans laquelle le traitement est effectué en étant à jeun au moins une heure avant chaque repas, dans laquelle les composants ci-dessus sont administrés simultanément, dans laquelle on administre 0,1 à 5 moles d'acide oxonique pour 1 mole de tégafur et dans laquelle on a une dégradation réduite de l'acide oxonique dans le tractus gastro-intestinal du patient.

**22.** Composition comprenant de l'acide oxonique et du tégafur destinée à être utilisée pour réduire la dégradation de l'acide oxonique dans le tractus gastro-intestinal d'un patient atteint d'un cancer susceptible d'être traité avec du 5-FU et en cours de traitement avec du tégafur, dans laquelle on administre au patient l'acide oxonique en même temps que ledit tégafur, dans laquelle l'administration s'effectue à jeun au moins une heure avant chaque repas et dans laquelle on administre 0,1 à 5 moles d'acide oxonique pour 1 mole de tégafur.

**23.** Composition destinée à être utilisée selon la revendication 21 ou 22, dans laquelle on administre deux fois par jour, simultanément et à jeun, le tégafur et l'acide oxonique.

**24.** Composition destinée à être utilisée selon la revendication 21 ou 22, dans laquelle on administre aussi en même temps un inhibiteur de la dihydropyrimidine déshydrogénase en quantité efficace pour potentialiser l'effet antitumoral du tégafur.

**25.** Composition destinée à être utilisée selon la revendication 24, dans laquelle l'inhibiteur de la dihydropyrimidine déshydrogénase est la 2,4-dihydroxy-5-chloropyridine.

**26.** Kit comprenant du tégafur, de la 2,4-di-hydroxy-5-chloropyridine, de l'acide oxonique ou un de ses sels pharmaceutiquement acceptables à des doses convenant à une administration deux fois par jour et destiné à être utilisé pour traiter le cancer chez un mammifère atteint d'un cancer susceptible d'être traité avec du 5-FU, le kit comprenant facultativement un agent antitumoral choisi dans le groupe constitué de la cisplatine, de la 1,3-bis(2-chloroéthyl)-1-nitrosourée, du docétaxel, de la doxorubicine, de l'épirubicine, de l'étoposide, du méthotrexate, de la mitomycine, de la gemcitabine, du carboplatine, du géfitinib, du pemetrexed, de l'Avastin, du cetuximab et du paclitaxel et le kit comprenant en outre un indicateur rappelant que le tégafur, la 2,4-dihydroxy-5-chloropyridine et l'acide oxonique ou un de ses sels pharmaceutiquement acceptables doivent être administrés à jeun au moins une heure avant chaque repas, dans lequel on administre 0,1 à 5 moles de 2,4-dihydroxy-5-chloropyridine et 0,1 à 5 moles d'acide oxonique ou d'un de ses sels pharmaceutiquement acceptables pour 1 mole de tégafur.

**27.** Composition pharmaceutique comprenant (A) une quantité thérapeutiquement efficace de tégafur, (B) un inhibiteur de la dihydropyrimidine déshydrogénase en quantité efficace pour potentialiser l'effet antitumoral et (C) de l'acide oxonique ou un de ses sels pharmaceutiquement acceptables en quantité efficace pour supprimer la toxicité gastro-intestinale destinée à potentialiser l'effet antitumoral du tégafur tout en réduisant la toxicité gastro-intestinale chez un patient atteint d'un cancer susceptible d'être traité avec du 5-FU et qui nécessite ce type de traitement, dans laquelle on administre 0,1 à 5 moles d'inhibiteur de la dihydropyrimidine déshydrogénase et 0,1 à 5 moles d'acide oxonique ou d'un de ses sels pharmaceutiquement acceptables pour 1 mole de tégafur, dans laquelle la composition pharmaceutique comprend facultativement un autre agent antitumoral choisi dans le groupe constitué du cisplatine, de la 1,3-bis(2-chloroéthyl)-1-nitrosourée, du docétaxel, de la doxorubicine, de l'épirubicine, de l'étoposide, du méthotrexate, de la mitomycine, de la gemcitabine, du carboplatine, du géfitinib, du pemetrexed, de l'Avastin, du cetuximab et du paclitaxel et dans laquelle la composition pharmaceutique est contenue dans un conditionnement comprenant un indicateur rappelant au patient que la composition pharmaceutique doit être administrée à jeun au moins une heure avant chaque repas.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5155113 A
- US 5116600 A
- US 5525603 A
- JP 49010510 A

**Non-patent literature cited in the description**

- **LOKICH et al.** *J. Clin. Oncl.,* 1989, vol. 7, 425-432
- **QUEBBEMAN, E. et al.** *J. Surg. Oncol.,* 1985, vol. 30, 60-65
- **HANSEN, R. et al.** *J. Surg. Oncol.,* 1989, vol. 40, 177-181
- **CABALLERO, G. A. et al.** *Cancer Treat. Rep.,* 1985, vol. 69, 13-15
- **BARBOUNIS, V. P. et al.** *Anticancer Res.,* 1989, vol. 9, 33-39
- **MOYNIHAN, T. et al.** *Am.J. Clin. Oncol.,* 1988, vol. I 1, 461-464
- **HUAN, S. et al.** *Cancer,* 1989, vol. 63, 419-422
- **HANSEN, R. et al.** *Am. J. Med. Sci.,* 1988, vol. 295, 91-93
- **HANSEN, R. et al.** *Urology,* 1991, vol. 37, 358-361
- **YAMAO, T. et al.** *Jpn. J. Clin. Oncol.,* 1995, vol. 25, 46-50
- **PETIT, E. et al.** *Cancer Res.,* 1988, vol. 48, 1676-1679
- **HARRIS, B. E. et al.** *Cancer Res.,* 1990, vol. 50, 197-201
- **METZGER, G. et al.** *Clin. Pharmacol. Ther.,* 1994, vol. 56, 190-201
- **FLEMING, R. A. et al.** *Cancer Res.,* 1992, vol. 52, 2899-2902
- **FLEMING, R. A. et al.** *Eur. J. Cancer,* 1993, vol. 29A, 740-744
- **ETIENNE, M. C. et al.** *J. Clin. Oncol.,* 1994, vol. 12, 2248-2253
- **BECK, A. et al.** *Eur. J. Cancer,* 1994, vol. 30A, 1517-1522
- **PETERS, G. J. et al.** *Eur. J. Cancer,* 1994, vol. 30A, 1408-1411
- **LU, Z. et al.** *Cancer Res.,* 1993, vol. 53, 5433-5438
- **ETIENNE, M. C. et al.** *J. Clin. Oncol.,* 1995, vol. 13, 1663-1670
- **SCHWARTZ et al.** *Cancer Res.,* 1979, vol. 39, 3095-3101
- **FOX, R. M. et al.** *Cancer Treat. Rev.,* 1979, vol. 6, 143-147
- **KROENER, J. F. et al.** *Cancer Treat. Rep.,* 1982, vol. 66, 1133-1137
- **HOWELL, S. B. et al.** *Cancer,* 1991, vol. 48, 1281-1289
- **WOOLLEY, P. V. et al.** *J. Clin. Oncol.,* 1985, vol. 3, 103-109
- **AHMANN, F. R. et al.** *Oncology,* 1986, vol. 43, 83-85
- **CLARK, P. I. et al.** *Eur. J. Surg. Oncol.,* 1985, vol. 11, 267-268
- **VLIET, W. et al.** *Clin. Pharm.,* 1989, vol. 8, 655-658
- **LOPRINZI, C. L. et al.** *Cancer,* 1990, vol. 65, 1879-1882
- **YAMADA Y. et al.** *Br. J. Cancer,* 01 September 2003, vol. 89 (5), 816-20
- **FUKUSHIMA M et al.** *Int. J. Mol. Med,* 2003, vol. 12, 839-844
- **MILANO et al.** *Eur. J. Cancer.,* 2000, vol. 36, 37-42
- **VAN GROENINGEN C.J. ; PETERS G.J. ; SCHOR-NAGEL J.H. ; GALL H. ; NOORDHUIS P. ; DE VRIES M. ; TURNER S.L. ; SWART M.S. ; PINEDO H.M. ; HANAUSKE A.R.** Phase I clinical and pharmacokinetic study of oral S-1 in patients with advanced solid tumors. *J. Clin. Oncol.,* 2000, vol. 18, 2772-2779
- **SHEPARD D.R. ; MANI S. ; KASTRISSIOS H. ; LEARNED-COUGHLIN S. ; SMITH D. ; MAGNUM P.E.S.M. ; JANISCH L. ; FLEMING G.F. ; SCHILSKY R.L. ; RATAIN M.J.** Estimation of the effect of food on the disposition of oral 5-fluorouracil in combination with eniluracil. *Cancer Chemother. Pharmacol.,* 2002, vol. 49, 398-402
- **REIGNER B. ; VERWEIJ J. ; DIRIX L. ; CASSIDY J. ; TWELVES C. ; ALLMAN D. ; WEIDEKAMM E. ; ROOS B. ; BANKEN L. ; UTOH M.** Effect of food on the pharmacokinetics of capecitabine and its metabolites following oral administration in cancer patients. *Clin. Cancer Res.,* 1998, vol. 4, 941-948

- **GODEFRIDUS J. PETERS ; PAUL NOORDHUIS ; CORNELIS J. VAN GROENINGEN ; GIUSEPPE GIACCONE ; ULBE HOLWERDA ; DAPHNE VOORN ; AD SCHRIJVERS ; JAN H. SCHORNAGEL ; JOS H. BEIJNEN ; PIERRE FU-MOLEAU.** The Effect of food on the pharmacokinetics of S-1 after single oral administration on patients with solid tumors. *Clinical Cancer Research,* 15 June 2004, vol. 10, 4072-4076
- **COLLINS J.M. ; DEDRICK R.L. ; KING F.G. ; SPEYER J.L. ; MYERS C.E.** Non-linear pharmacokinetic models for 5-fluorouracil in man. *Clin. Pharmacol. Therap.,* 1980, vol. 28, 235-246
- **PETERS G.J. ; NOORDHUIS P. ; VAN KUILENBURG A.B.P. ; SCHORNAGEL J.H. ; GALL H. ; TURNER S.L. ; SWART M.S. ; VOORN D. ; VAN GENNIP A.H. ; WANDERS J.** Pharmacokinetics of S-1, an oral formulation of tegafur, oxonic acid and 5-chloro-2,4-dihydroxypyridine (molar ratio 1:0.4:1) in patients with solid tumors. *Cancer Chemother. Pharmacol.,* 2003, vol. 38, 1-12
- **HIRATA K. ; HORIKOSHI N. ; OKAZAKI M. ; DENNO R. ; SASAKI K. ; NAKANO Y. ; ISHIZUKA H. ; YAMADA Y. ; UNO S. ; TAGUCHI T.** Pharmacokinetic study of S-1, a novel oral fluorouracil anti-tumor drug. *Clin. Cancer Res.,* 1999, vol. 5, 2000-2005
- **IKEDA M. ; FURUKAWA H. ; IMAMURA H. ; SHIMIZU J. ; ISHIDA H. ; MASUTANI S. ; TATSUTA M. ; KAWASAKI T. ; SATOMI T.** Pharmacokinetic study of S-1, a novel oral fluorouracil antitumor agent in animal model and in patients with impaired renal function. *Cancer Chemother. Pharmacol.,* 2002, vol. 50, 25-32
- **OHTSU A. ; BABA H. ; SAKATA Y.** Phase II study of S-1, a novel oral fluoropyrimidine derivative, in patients with metastatic colorectal carcinoma. S-1 cooperative Colorectal Carcinoma Study Group. *Br. J. Cancer,* 2000, vol. 83, 141-145
- **VAN DEN BRANDE J. ; SCHOFFSKI P. ; SCHELLENS J.H. ; ROTH AD ; DUFFAUD F. ; WEIGANG-KOHLER K. ; REINKE F. ; WANDERS J. ; DE BOER R.F. ; VERMORKEN J.B.** EORTC Early Clinical Studies Group early phase II trial of S-1 in patients with advanced or metastatic colorectal cancer. *Br. J. Cancer,* 2003, vol. 88, 648-53
- **SHIRASAKA T. ; SHIMAMOTO Y. ; FUKUSHIMA M.** Inhibition by oxonic acid of gastrointestinal toxicity of 5-fluorouracil without loss of its antitumor activity in rats. *Cancer Res.,* 1993, vol. 53, 4004-4009
- *N. Engl. J. Med.,* 22 October 1987, vol. 317 (17), 1098
- *Arch. Int. Med.,* 1916, vol. 17, 863-71
- *J. Clin. Anesth.,* 1992, vol. 4 (1), 4-10
- *The Journal of Pediatrics,* 1978, vol. 93 (1), 62-66
- *Cancer Chemother. Rep.,* 1970, vol. 54, 225-35
- *Nippon Eiseigaku Zasshi,* 1968, vol. 5, 443-50